(19) Europäisches Patentamt
European Patent Office
Office européen des brevets

(11) Veröffentlichungsnummer: **0 303 570**
**A2**

(12) # EUROPÄISCHE PATENTANMELDUNG

(21) Anmeldenummer: **88810532.7**

(22) Anmeldetag: **03.08.88**

(51) Int. Cl.4: **C 07 D 213/40**
A 01 N 47/42

(30) Priorität: **12.08.87 CH 3099/87**

(43) Veröffentlichungstag der Anmeldung:
**15.02.89 Patentblatt 89/07**

(84) Benannte Vertragsstaaten:
**CH DE FR GB IT LI**

(71) Anmelder: **CIBA-GEIGY AG**
**Klybeckstrasse 141**
**CH-4002 Basel (CH)**

(72) Erfinder: **Gsell, Laurenz, Dr.**
**Maiengasse 56**
**CH-4056 Basel (CH)**

(54) **Substituierte Isothioharnstoffe.**

(57) Neue, substituierte N-Pyridylmethyl-N′-cyano-isothioharnstoffe der Formel

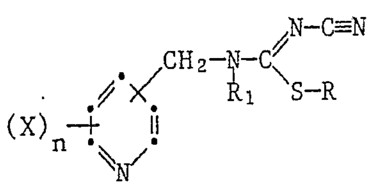

worin
R $C_1$-$C_4$-Alkyl oder Benzyl;
$R_1$ $C_1$-$C_4$-Alkyl;
X Halogen; und
n eine Zahl 0, 1, 2 oder 3
bedeuten.

Es werden beschrieben Verfahren zur Herstellung dieser Verbindungen, die entsprechenden Ausgangs- und Zwischenprodukte und die Verwendung der neuen Verbindungen in der Schädlingsbekämpfung, insbesondere zur Bekämpfung von Insekten und Vertretern der Ordnung Akarina, speziell von Schadinsekten in Reiskulturen, sowie als Ausgangsprodukte für die Synthese neuer Schädlingsbekämpfungsmittel.

EP 0 303 570 A2

Bundesdruckerei Berlin

## Beschreibung

### Substituierte Isothioharnstoffe

Die vorliegende Erfindung betrifft neue, substituierte N-Pyridylmethyl-N'-cyano-isothioharnstoffe, Verfahren zu ihrer Herstellung und ihre Verwendung in der Schädlingsbekämpfung sowie als Ausgangsprodukte für die Synthese neuer Schädlingsbekämpfungsmittel.

Die Erfindung bezieht sich auf neue Verbindungen der Formel I

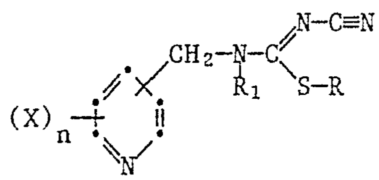

(I),

worin
R $C_1$-$C_4$-Alkyl oder Benzyl;
$R_1$ $C_1$-$C_4$-Alkyl;
X Halogen; und
n eine Zahl 0, 1, 2 oder 3
bedeuten.

Bevorzugt werden erfindungsgemäss Verbindungen der Formel I, worin
R Methyl, Ethyl oder Benzyl;
$R_1$ Methyl;
X Chlor; und
n eine Zahl 0, 1 oder 2
bedeuten.

Weiterhin bevorzugt werden erfindungsgemäss Verbindungen der Formel I, worin
R und $R_1$ Methyl; und
n die Zahl 0
bedeuten.

Von besonderem Interesse sind solche erfindungsgemässen Verbindungen der Formel I, worin der Pyridylrest ein Pyrid-3-yl- oder Pyrid-4-yl-Rest ist.

Unter dem Begriff Alkyl selbst oder als Bestandteil eines anderen Substituenten sind geradkettige oder verzweigte und je nach Zahl der angegebenen Kohlenstoffatome im Rahmen der vorliegenden Erfindung beispielsweise folgende Gruppen zu verstehen: Methyl, Ethyl, Propyl, Butyl, sowie ihre Isomeren, wie Isopropyl, Isobutyl, tert.-Butyl, sek.-Butyl.

Unter dem Begriff Halogen im Rahmen der vorliegenden Erfindung ist Fluor, Chlor und Brom zu verstehen, vorzugsweise Fluor und Chlor.

Der vorliegende Erfindungsgegenstand umfasst auch die Salze, insbesondere die pflanzenphysiologisch unbedenklichen Salze, der Verbindungen der Formel I. Als derartige Salze mit organischen und anorganischen Säuren kommen z.B. die folgenden in Betracht: Chloride, Bromide, Jodide, Sulfate, Hydrogensulfate, Chlorate, Perchlorate, Rhodanide, Nitrate, Phosphate, Hydrogenphosphate, Tetrafluorborate, Formiate, Acetate, Trichloracetate, Trifluoracetate, Phenylsulfonate, Oxalate, Malonate, Succinate, Malate, Tartrate oder Citrate.

Die Verbindungen der Formel I können in an sich bekannter Weise (vgl. z.B. JP-Patentanmeldung Kokai Nr. 62-234064; J.B. Hendrickson et al, "Organic Chemistry", McGraw Hill Book Co., 1970, S. 378 - 382) hergestellt werden, indem man eine Verbindung der Formel II

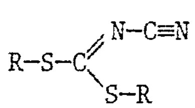

(II)

mit einer Verbindung der Formel III

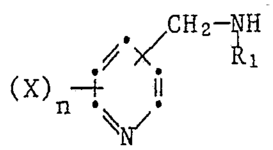

(III);

umsetzt; wobei in den Formeln II und III R, $R_1$, X und n die vorstehend angegebenen Bedeutungen haben. Bei dieser Reaktion ist einer der Reste -S-R eine unter den Reaktionsbedingungen abspaltbare Abgangsgruppe.

Die zu den erfindungsgemässen Verbindungen der Formel I führenden obigen Verfahrensvarianten werden vorzugsweise in einem Lösungsmittel vorgenommen. Geeignete Lösungsmittel sind z.B. aromatische Kohlenwasserstoffe, wie Benzol, Toluol und Xylol; Ketone, wie Aceton, Cyclohexanon und Methyläthylketon; Aether, wie Tetrahydrofuran, Dioxan und Diäthyläther; halogenierte Kohlenwasserstoffe, wie Chloroform, Tetrachlorkohlenstoff und Chlorbenzol; Alkohole, wie Aethanol und Propanol; Ester aliphatischer Säuren, wie Aethylacetat; aliphatische Amide, wie Dimethylformamid und Dimethylacetamid; Dimethylsulfoxid, Acetonitril und andere Lösungsmittel, die die Reaktion nicht beeinträchtigen. Diese Lösungsmittel können auch als Gemische verwendet werden. Die Reaktionstemperatur kann in einem weiten Bereich von -10 bis +150°C liegen. Bevorzugt wird ein Temperaturbereich von etwa 20° bis 80°C.

Die Ausgangsverbindungen der Formeln II und III sind bekannt oder sie können, falls sie neu sind, analog bekannten Methoden erhalten werden. So sind die N-Cyanothioiminocarbonate der Formel II, sowie ihre Herstellung aus Chem. Ber. 100, 2604-15 (1967); J. Hetero. Chem. 19, 1205-6 (1982) und der JP Patentanmeldung SHO 61-76044 bekannt. Die Picolylamin-Verbindungen der Formel III sind ebenfalls bekannt oder sie können z.B. analog Tetrahedron Letters Vol. 26, 5863 (1985) oder Agr. Biol. Chem. 32, 747 (1968) hergestellt werden.

N-Picolyl-N′-cyano-isothioharnstoffe, ihre Herstellung sowie ihre Verwendung als Pharmazeutika sind bereits in der JP-Patentanmeldung SHO 61-76044 beschrieben worden. Demgegenüber unterscheiden sich die erfindungsgemässen Verbindungen der Formel I strukturell durch die Alkylsubstitution am N-Atom ($R_1$ in Formel I).

Es wurde nun überraschenderweise gefunden, dass die erfindungsgemässen Guanidin-Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tieren befallenden Schädlingen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Mit Hilfe der erfindungsgemässen Verbindungen der Formel I können vor allem pflanzenschädigende Insekten, speziell pflanzenschädigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische, vor allem aber durch Kontakt-Wirkung gegen saugende Insekten, insbesondere gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die Verbindungen der Formel I zeichnen sich weiterhin durch eine gute Wirkung gegen larvale Insektenstadien und Nymphen, speziell von fressenden Schadinsekten, aus. Insbesondere können die Verbindungen der Formel I mit ausgezeichnetem Erfolg gegen pflanzenschädigende Zikaden, speziell in Reis-Kulturen, eingesetzt werden. In diesem Zusammenhang wird auf die geringe Fisch-Toxizität der erfindungsgemässen Verbindungen hingewiesen.

Die Verbindungen eignen sich ferner zur Bekämpfung von Ektoparasiten, z.B. Lucilia sericata, sowie von Zecken an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte, Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff, bzw. Kombinationen dieser Wirkstoffe mit anderen Insektiziden oder Akariziden, und gegebenenfalls einen festen oder flüssigen Zusatzstoff enthaltenden Mittel,

Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctyl phthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxy polyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten - auf das Gewicht bezogen -in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen, z.B. 0,1 bis 1000 ppm.

Die Mittel können auch weitere Zusätze wie Stabilisatoren, Entschäumer, Viskositätsregulatoren,

Bindemittel, Haftmittel sowie Dünger oder andere Wirkstoffe zur Erzielung spezieller Effekte enthalten.

Die erfindungsgemässen Isothioharnstoff-Verbindungen der Formel I sind auch geeignet zur Verwendung der Ausgangs- bzw. Zwischenprodukte für die Herstellung pestizid, insbesondere insektizid, wirksamer Guanidin-Ver bindungen. Ein weitere Gegenstand der vorliegenden Erfindung ist daher die Verwendung einer Verbindung der Formel I zur Herstellung von pestizid wirksamen Guanidin-Verbindungen der Formel IV und deren Tautomeren

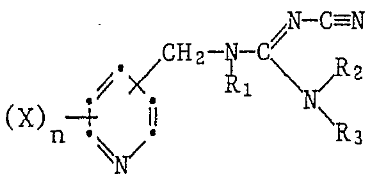

(IV),

worin
$R_1$ und $R_2$ unabhängig voneinander $C_1$-$C_4$-Alkyl,
$R_3$ Wasserstoff, $C_1$-$C_4$-Alkyl, Benzyl oder Picolyl; oder
$R_2$ und $R_3$ zusammen einen Rest $-(CH_2)-_4$ oder $-(CH_2)-_5$;
X Halogen; und
n eine Zahl 0, 1, 2 oder 3;
bedeuten, durch Umsetzung einer Verbindung der Formel I mit einer Verbindung der Formel V

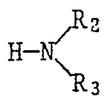

(V),

worin $R_2$ und $R_3$ die vorstehend angegebenen Bedeutungen haben.

Beispiel 1: Herstellung von N-Pyrid-3-ylmethyl-N-methyl-N′-cyano-S-methylisothio-harnstoff

Es werden 5,1 g Dimethyl-N-cyanothioiminocarbonat 4,3 g 3-Methylaminomethylpyridin und 10,6 g Ammoniumacetat in 20 ml Aethanol während 8 Std. am Rückfluss gehalten. Nach Erkalten des Reaktionsgemisches werden die Salze entfernt und die Lösungsmittel destilliert. Nach der Reingung durch Chromatographie wird das Produkt als helles Oel isoliert. Auf diese Weise wird die Titelverbindung der Formel

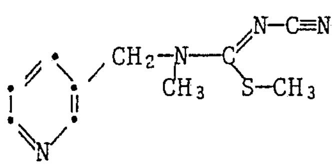

mit einer Refraktion von $n_D^{20}$= 1,6098 (Verbindung Nr. 1) erhalten.

Wie vorstehend aufgeführt werden auch die folgenden Verbindungen der Formel I hergestellt:

| Verb. Nr. | Stellung Pyridyl | R | $R_1$ | X | n | phys. Data |
|---|---|---|---|---|---|---|
| 2 | 4- | 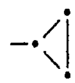 | H | - | 0 | F = 125 - 26°C |
| 3 | 3- | $-C_3H_7(i)$ | H | - | 0 | F = 107 - 109,5°C |
| 4 | 4- | $-CH_3$ | $-C_3H_7(n)$ | - | 0 | $n_D^{23}$ = 1,5951 |
| 5 | 3- | $-CH_3$ | 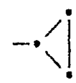 | - | 0 | $n_D^{24}$ = 1,6105 |

Wie vorstehend angegeben sind auch die folgenden Verbindungen der Formel I erhältlich:

| Stellung Pyridyl | R | $R_1$ | X | n |
|---|---|---|---|---|
| 3- | $-CH_3$ | $-C_2H_5$ | - | 0 |
| 3- | $-CH_3$ | $-C_4H_9(n)$ | - | 0 |
| 2- | $-CH_3$ | $-CH_3$ | - | 0 |
| 2- | $-CH_3$ | $-C_2H_5$ | - | 0 |
| 4- | $-CH_3$ | $-CH_3$ | - | 0 |
| 4- | $-CH_3$ | $-C_4H_9(n)$ | - | 0 |

Beispiel 2: Herstellung von N-Pyrid-3-ylmethyl-N-methyl-N′-cyano-N″-n-butylguanidin

Es werden 4,5 g der nach Beispiel 1 hergestellten Verbindung Nr. 1 zusammen mit 2 ml n-Butylamin und 20 ml Aethanol während 16 Stunden am Rückfluss gehalten. Nach Abdestillieren der flüchtigen Anteile am Rotationsverdampfer wird der Rückstand mittels Chromatographie an einer Kieselgel-Säule unter Elution mit Dichlormethan/Methanol (90/10 Vol.-%) gereinigt. Man erhält die Titelverbindung der Formel

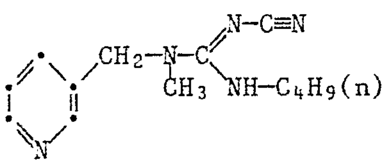

als hellgelbes Oel ($n_D^{22}$ = 1,5598).

Beispiel 3: Formulierungen für flüssige Wirkstoffe der Formel I gemäss Beispiel 1 oder Kombinationen dieser Wirkstoffe mit anderen Insektiziden oder Akariziden (% = Gewichtsprozent):

3.1. Emulsions-Konzentrate

| | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

3.2. Lösungen

| | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

3.3. Granulate

| | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 3.4. Stäubemittel | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I gemäss Beispiel 1 oder Kombinationen dieser Wirkstoffe mit andern Insektiziden oder Akariziden (% = Gewichtsprozent):

| 3.5. Spritzpulver | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - 2 % | - | |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

3.6. Emulsions-Konzentrat

Wirkstoff oder Wirkstoffkombination 10 %
Octylphenolpolyäthylenglykoläther (4-5 mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

3.7. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

3.8. Extruder-Granulat

Wirkstoff oder Wirkstoffkombination 10 %
Na-Ligninsulfonat 2 %
Carboxymethylcellulose 1 %
Kaolin 87 %

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

3.9. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granuate.

3.10 Suspensions-Konzentrat

Wirkstoff oder Wirkstoffkombination 40 %
Aethylenglykol 10 %
Nonylphenolpolyäthylenglykoläther (15 Mol AeO) 6 %
Na-Ligninsulfonat 10 % Carboxymethylcellulose 1 %
37 %ige wässrige Formaldehyd-Lösung Silikonöl in Form einer 75 %igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Insektizide Kontaktwirkung: Aphis craccivora

In Töpfen gezogene Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe besprüht. Man verwendet pro Test-Verbindung zwei Pflanzen, und eine Auswertung der erzielten Abtötungsrate erfolgt nach weiteren 24 Stunden.

Verbindungen der Formel I gemäss Beispiel 1 zeigen gute Wirksamkeit (Mortalität) in diesem Test.

Beispiel 5: Insektizide systemische Wirkung: Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25%igen Spritzpulver) jeweils in einer Konzentration von 400 ppm direkt auf der Erde in den Töpfen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt-oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet. Der Versuch wird bei 25°C und 70%

relativer Luftfeuchtigkeit durchgeführt.

Die Verbindungen der Formel I gemäss Beispiel 1 zeigen gute Wirkung in obigem Test.

Beispiel 6: Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Man verwendet pro Ansatz zwei Pflanzen. Eine Auswertung der erzielten Abtötungsrate erfolgt 48 Stunden nach Applikation. Der Versuch wird bei 20-22° C und 60 % relativer Luftfeuchtigkeit durchgeführt.

Die Verbindungen der Formel I gemäss Beispiel 1 zeigen gute Wirkung in obigem Test.

Beispiel 7: Insektizide systemische Wirkung: Myzus persicae

Bewurzelte Kohlpflanzen im 4- bis 5-Blatt-Stadium werden in Töpfe, welche 60 ccm Erde enthalten, verpflanzt. Anschliessend werden 50 ml einer wässrigen Formulierung der zu prüfenden Verbindung I (erhalten aus einem 25%igen Spritzpulver) jeweils in einer Konzentration von 400 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25° C und 60 % relativer Luftfeuchtigkeit durchgeführt.

Die Verbindungen der Formel I gemäss Beispiel 1 zeigen gute Wirkung in obigem Test.

Beispiel 8: Insektizide Blattpenetrations-Wirkung: Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffs in einer Konzentration von 400 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60 % relativer Luftfeuchtigkeit durchgeführt. Die Auswertung auf %-Mortalität erfolgt 48 Stunden nach Wirkstoffapplikation.

Verbindungen der Formel I gemäss Beispiel 1 zeigen gute Wirkung in obigem Test.

Beispiel 9: Insektizide Wirkung (systemisch-Wasser): Aphis craccivora

Erbsenkeimlinge, die 24 Stunden vor Beginn des Versuches mit den Blattläusen infestiert worden waren, werden in 20 ml einer wässrigen Brühe gestellt, die 400 ppm des zu prüfenden Wirkstoffes enthält. Die wässrige Brühe wird aus einem Emulsionskonzentrat oder einer benetzbaren Pulverzubereitung des betreffenden Wirkstoffes hergestellt und befindet sich in einem Gefäss, dass mit einem Löcher aufweisenden Plastikdeckel abgeschlossen ist. Die Wurzel der infestierten Erbsenpflanze wird jeweils durch ein Loch in dem Plastikdeckel in die Brühe geschoben. Das Loch wird dann mit Watte abgedichtet, um die Pflanze zu fixieren und einen eventuellen Einfluss der Gasphase aus der Brühe auszuschalten.

Der Versuch wird bei 20° C und 60° C relativer Luftfeuchtigkeit durchgeführt. Nach zwei Tagen wird auf die Anzahl der nicht mehr saugfähigen Testtiere im Vergleich zu unbehandelten Kontrollen bonitiert. Damit wird festgestellt, ob der über die Wurzel aufgenommene Wirkstoff, die Blattläuse an den oberen Pflanzenteilen abtötet.

Verbindungen der Formel I gemäss Beispiel 1 zeigen im obigen Versuch eine gute systemische Wirkung gegen Insekten der Spezies Aphis craccivora.

Beispiel 10: Frassgift- und Kontakt-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages erfolgt die Besiedlung jeder Pflanze

mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I gemäss Beispiel 1 zeigen gute Wirkung in diesem Test gegen Nilaparvate lugens.

Beispiel 11: Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 400 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wurde dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20°C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen der Formel I gemäss Beispiel 1 zeigen im obigem Test gute Wirkung (Mortalität) gegen Nilaparvata lugens.

Beispiel 12: Insektizide Frassgift- und Kontakt-Wirkung

Ca. 25 cm hohe eingetopfte Baumwollpflanzen werden mit wässrigen Wirkstoffemulsionen besprüht, die den Wirkstoff in einer Konzentration von 800 ppm enthalten.

Nach dem Antrocknen des Sprühbelages werden die Baumwollpflanzen mit Spodoptera littoralis- bzw. Heliothis virescens-Larven im ersten larvalen Stadium besiedelt. Der Versuch wird bei 24°C und etwa 60 % relativer Luftfeuchtigkeit durchgeführt. Nach 120 Stunden wird die %-Mortalität der Test-Insekten gegenüber unbehandelten Kontrollen bestimmt.

Verbindungen der Formel I gemäss Beispiel 1 zeigen gute Wirkung (Mortalität) in diesem Test.

Beispiel 13: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Verbindungen der Formel I gemäss Beispiel 1 zeigen in diesem Test gute Wirkung.

**Patentansprüche**

1. Verbindung der Formel I

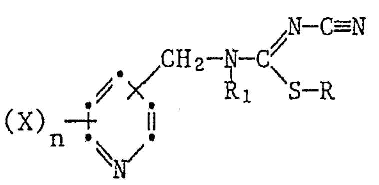

(I),

worin
R $C_1$-$C_4$-Alkyl oder Benzyl;
$R_1$ $C_1$-$C_4$-Alkyl;
X Halogen; und
n eine Zahl 0, 1, 2 oder 3
bedeuten.

2. Verbindung der Formel I gemäss Anspruch 1, dadurch gekennzeichnet, dass
R Methyl, Ethyl oder Benzyl;
$R_1$ Methyl;
X Chlor; und
n eine Zahl 0, 1 oder 2
bedeuten.

3. Verbindung der Formel I gemäss Anspruch 2, dadurch gekennzeichnet, dass
R und $R_1$ Methyl; und
n die Zahl 0
bedeuten.

4. Verbindung der Formel I gemäss einem der Ansprüche 1 bis 3, dadurch gekennzeichnet, dass der Pyridylrest ein Pyrid-3-yl- oder Pyrid-4-yl-Rest ist.

5. Verbindung gemäss Anspruch 4 der Formel

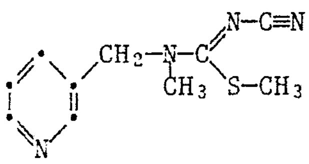

.

6. Verbindung gemäss Anspruch 4 der Formel

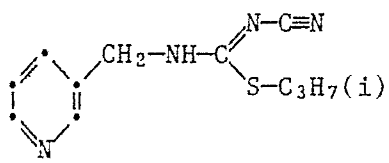

7. Verbindung gemäss Anspruch 4 der Formel

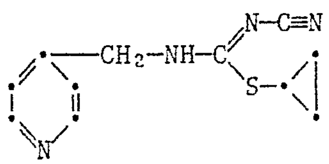

8. Verfahren zur Herstellung einer Verbindung der Formel I gemäss Anspruch 1, dadurch gekennzeichnet, dass man eine Verbindung der Formel II

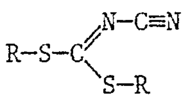

(II)

mit einer Verbindung der Formel III

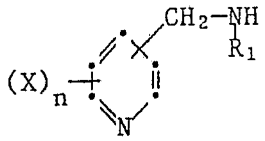

(III);

umsetzt; wobei in den Formeln II und III R, $R_1$, X und n die in Anspruch 1 angegebenen Bedeutungen haben.

9. Verwendung einer Verbindung der Formel I gemäss Anspruch 1 zur Herstellung von Verbindungen der Formel IV und deren Tautomeren

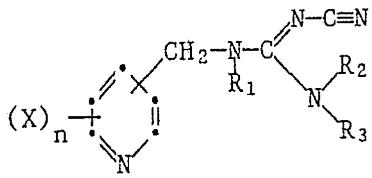

(IV),

worin
$R_1$ und $R_2$ unabhängig voneinander $C_1$-$C_4$-Alkyl,
$R_3$ Wasserstoff, $C_1$-$C_4$-Alkyl, Benzyl oder Picolyl; oder
$R_2$ und $R_3$ zusammen einen Rest $-(CH_2)_4$ oder $-(CH_2)_5$;
X Halogen; und
n eine Zahl 0, 1, 2 oder 3
bedeuten, durch Umsetzung einer Verbindung der Formel I mit einer Verbindung der Formel V

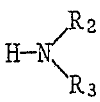

(V),

worin $R_2$ und $R_3$ die vorstehend angegebenen Bedeutungen haben.

10. Verwendung einer Verbindung der Formel I gemäss einem der Ansprüche 1 bis 7 zur Bekämpfung pflanzenschädigender Insekten und Vertretern der Ordnung Akarina.

11. Verwendung gemäss Anspruch 10 zur Bekämpfung von Schadinsekten in Reiskulturen.

12. Mittel zur Bekämpfung von Schadinsekten und Schädlingen der Ordnung Akarina enthaltend als Aktive Komponente mindestens eine Verbindung der Formel I gemäss einem der Ansprüche 1 bis 7.

13. Verfahren zur Bekämpfung von schädlichen Insekten und Vertretern der Ordnung Akarina, dadurch gekennzeichnet, dass man die Schädlinge, bzw. deren verschiedene Entwicklungsstadien oder deren Aufenthaltsort, mit einer pestizid wirksamen Menge einer Verbindung der Formel I gemäss einem der Ansprüche 1 bis 7 oder mit einem Mittel gemäss Anspruch 12 enthaltend neben Zusatz- und Trägerstoffen eine pestizid wirksame Menge dieser Verbindung, in Kontakt bringt oder behandelt.